# EUROPÄISCHE PATENTANMELDUNG

(11) **EP 4 474 032 A1**
(43) Veröffentlichungstag der Anmeldung: **11.12.2024**
(21) Anmeldenummer: 23177928.1
(22) Anmeldetag: 07.06.2023
(51) Int. Cl.: B01D 5/00, C07C 29/151, C07C 31/04

(54) **WECHSELLASTBETRIEB EINER METHANOLANLAGE**

(71) Anmelder: L'AIR LIQUIDE, SOCIETE ANONYME POUR L'ETUDE ET L'EXPLOITATION DES PROCEDES GEORGES CLAUDE, 75007 Paris (FR)
(72) Erfinder: Peña, Vincent, 60439 Frankfurt am Main (DE); Oelmann, Tobias, 60439 Frankfurt am Main (DE); Gronemann, Veronika, 60439 Frankfurt am Main (DE); Strozyk, Michael, 60439 Frankfurt am Main (DE)
(74) Vertreter: Air Liquide

(57) **Zusammenfassung**

Verfahren zur Synthese von Methanol, wobei ein Feedgasstrom umfassend zumindest ein erstes Edukt und ein zweites Edukt einer Synthesereaktoranordnung (1) zugeführt wird, wobei in der Synthesereaktoranordnung (1) mit dem ersten Edukt und dem zweiten Edukt Methanol erzeugt wird, wobei in zumindest einem ersten Fall einer Reduzierung einer Stoffmengenströmungsrate des Feedgasstroms eine Erzeugungsrate des Methanols zumindest zeitweise reduziert wird, indem ein Anteil des ersten Eduktes in dem Feedgasstrom reduziert wird. Ein erstes Edukt umfasst Kohlenstoffdioxid und/oder Kohlenstoffmonoxid und das zweite Edukt umfasst Wasserstoff.

## Beschreibung

Die vorliegende Erfindung betrifft ein Verfahren und eine Vorrichtung zur Synthese von Methanol, insbesondere im Wechsellastbetrieb.

Konventionelle Anlagen zur Synthese von Methanol sind dahingehend ausgelegt, dass sie kontinuierlich bei einer konstanten Auslastung produzieren. Änderungen in der Auslastung sind im Normalbetrieb minimal. Dies wird durch die kontinuierliche Versorgung mit Feedgas ermöglicht. Lastwechsel und Prozessanpassungen zwischen definierten, stationären Zuständen erfolgen sehr langsam über Stunden bis Tage, um die Methanolanlage und den Katalysator zu schützen.

Der planmäßige Wechsellastbetrieb des Syntheseloops mit einer Synthesereaktoranordnung ist grundsätzlich nicht vorgesehen. Insbesondere ist der Betrieb unter sehr geringer Teillast nicht üblich. Minimallasten im Produktionsbetrieb liegen typischerweise bei mindestens 50% bis 70% der Anlagenkapazität. Der Teillastfall kann für längere Zeiträume mit reduzierter Gasverfügbarkeit über mehrere Wochen oder Monate im Winter vorgesehen sein.

Lastwechsel und Prozessanpassungen zwischen definierten stationären Zuständen erfolgen sehr langsam über Stunden bis Tage, um das Equipment und die Katalysatoren der Methanolanlage vor Beschädigung zu schützen.

Gewöhnliches Feedgas aus Industrieprozessen enthält hauptsächlich Wasserstoff, Kohlenstoffmonoxid und Kohlenstoffdioxid.

Die Synthese von Methanol kann grundsätzlich mit Wasserstoff durchgeführt werden, welcher von einem Elektrolyseur produziert wird. Der Elektrolyseur kann unter anderem mit erneuerbaren Energien betrieben werden. Dieser Betrieb mit erneuerbaren Energien ist vor die Herausforderung gestellt, dass die Wasserstoffproduktion mit Fluktuationen beaufschlagt ist. Die Fluktuationen der produzierten Wasserstoffmenge aus erneuerbaren Energien hängen beispielsweise von der verfügbaren Solarenergie und der verfügbaren Windenergie ab. Beispielsweise sinkt die Solarenergie in der Nacht ab oder die Windkraft sinkt während einer Sommerflaute. Typische Lastwechsel können sich hier innerhalb weniger Sekunden bis Minuten abspielen. Änderungen der Last des Elektrolyseurs sind in einer ähnlichen Größenordnung möglich. Lastwechsel bei fluktuierenden erneuerbaren Energien und des Elektrolyseurs sind jedoch um eine Größenordnung schneller als der Lastwechsel der Synthesereaktoranordnung. Des Weiteren ist der zulässige Betriebsbereich der Synthesereaktoranordnung stärker eingeschränkt als der des Elektrolyseurs und der erneuerbaren Energien. Die Synthesereaktoranordnung für die Synthese von Methanol verhält sich hierbei träge.

Im stationären Dauerbetrieb läuft der Syntheseloop mit der Synthesereaktoranordnung unter annähernd konstanten Druck- und Temperaturbedingungen, was dementsprechend einen gleichmäßigen Betriebszustand für Apparate, Maschinen und den Katalysator der Methanolanlage bedeutet. Der Druck im Syntheseloop kann über ein Druckhalteventil konstant gehalten werden, welches Inertkomponenten und unreagierte Edukte sowie nicht abgeschiedene Produkte ablässt, sobald der Syntheseloopdruck einen bestimmten Druck übersteigt. Herausfordernd ist bei einem fluktuierenden Betrieb, also bei nicht konstanter Zufuhr der Edukte, den Betriebsdruck im Syntheseloop aufrechtzuhalten und einen hohen Umsatz der Edukte in der Synthesereaktoranordnung zu ermöglichen.

Eine Möglichkeit den Umsatz der Edukte zu erhöhen ist es, unreagierte Edukte wieder im Syntheseloop rezirkulieren zu lassen (Recycle). Zudem kann durch den Recycle erreicht werden, dass sich in den Eduktgasströmen enthaltenes Inertgas im Syntheseloop akkumuliert und den Druck des Syntheseloops stabilisiert. Bei geringen Inertgasanteilen in den Eduktgasströmen kann diese Stabilisierung des Druckes zu langsam sein, um den Fluktuationen eines zweiten Eduktstromes zu folgen und somit nicht oder nur unzureichend nutzbar sein. Auch ist dieser Effekt nicht nutzbar, wenn der Anteil an Inerten in den Edukstömen sehr gering oder gar Null ist.

Bei einem Wechsel von Volllast im Normalbetrieb in den Teillastbetrieb (Verringerung der Eduktgasströme) sinkt ohne zusätzliche Maßnahmen wie z.B. Veränderung des Recycle-Mengenstromes der Druck im Syntheseloop. Ursächlich ist hier, dass der Syntheseloop zunächst noch eduktreiches rezirkuliertes Recyclegas enthält, welches im Reaktor reagiert und Methanol bildet. Das Methanol wird im Abscheider abgeschieden. Dies führt dazu, dass die Gasphase im Syntheseloop an Stoffmenge verliert. Der Partialdruck der gasförmigen Edukte sinkt ab. Der Druck des konstanten Volumens des Syntheseloops sinkt folglich auch ab. Das Druckhalteventil kann den Druckabfall nicht verhindern, da dies nur einen erhöhten Druck durch Öffnung des Ventils ablassen kann.

Sinkt der Feedgasstrom durch Fluktuationen bedingt durch beispielsweise einen Elektrolyseur, welcher mit erneuerbaren Energien betrieben wird, rapide innerhalb von Sekunden bis Minuten ab, kann der Druck im Methanolreaktor nicht schnell genug stabilisiert werden. Ein rapider Anstieg des Feedgasstroms kann hingegen über das Druckhalteventil stabilisiert werden, um einen Betriebsdruck für eine Teillast oder Volllast nicht zu überschreiten.

Hiervon ausgehend liegt der vorliegenden Erfindung die Aufgabe zugrunde, einen Wechsellastbetrieb in der Synthese von Methanol zu ermöglichen.

Diese Aufgabe wird gelöst mit dem Verfahren und der Vorrichtung gemäß den unabhängigen Ansprüchen. Weitere vorteilhafte Ausgestaltungen der Erfindung sind in den abhängig formulierten Ansprüchen angegeben. Die in den abhängig formulierten Ansprüchen einzeln aufgeführten Merkmale sind in technologisch sinnvoller Weise miteinander kombinierbar und können weitere Ausgestaltungen der Erfindung definieren. Darüber hinaus werden die in den Ansprüchen angegebenen Merkmale in der Beschreibung näher präzisiert und erläutert, wobei weitere bevorzugte Ausgestaltungen der Erfindung dargestellt werden.

Erfindungsgemäß wird ein Verfahren zur Synthese von Methanol vorgestellt, wobei ein Feedgasstrom zumindest ein erstes Edukt und ein zweites Edukt umfasst, welches einer Synthesereaktoranordnung zugeführt wird. In der Synthesereaktoranordnung wird mit dem ersten Edukt und dem zweiten Edukt Methanol erzeugt. In zumindest einem ersten Fall einer Reduzierung einer Stoffmengenströmungsrate des Feedgasstroms wird eine Erzeugungsrate des Methanols zumindest zeitweise reduziert, indem ein Anteil des ersten Eduktes in dem Feedgasstrom reduziert wird.

Das erste Edukt und das zweite Edukt können zu einem Feedgasstrom gemischt werden. Der Feedgasstrom wird der Synthesereaktoranordnung zugeführt. In der Synthesereaktoranordnung können das erste Edukt und das zweite Edukt zu Methanol als einem Produkt und optional zu einem oder mehreren Nebenprodukten reagieren.

In der Synthesereaktoranordnung sind das erste Edukt und das zweite Edukt vorzugsweise gasförmig.

Die Synthesereaktoranordnung kann aus einem Reaktorsystem geeigneter Reaktoren bestehen. Die Synthesereaktoranordnung umfasst vorzugsweise einen Reaktor, bevorzugt mehrere parallel oder in Serie geschaltete Reaktoren. Die Synthesereaktoranordnung kann insbesondere durch einen wassergekühlten und/oder gasgekühlten Reaktor gebildet sein.

Der in der Gasphase in der Synthesereaktoranordnung befindliche Stoffmengenstrom kann aufgrund der Stöchiometrie der Methanolbildungsreaktion abnehmen. Das heißt die "Anzahl" der Moleküle in der Gasphase nimmt durch die Reaktion ab, wodurch der Druck sinkt. Durch einen verringerten Stoffmengenstrom in der Gasphase kann der Druck im Syntheseloop absinken, sofern nicht mit dem in den Syntheseloop eintretenden Feedgas die aus der Gasphase verlorene Stoffmenge wieder zugeführt wird.

Der Stoffmengenstrom kann beispielsweise in kmol pro Zeiteinheit angegeben werden.

Aufgrund der Stöchiometrie der Methanolbildungsreaktion ist das erste Edukt im Feedgas zum Syntheseloop vorzugsweise in einem niedrigeren Anteil vorhanden als das zweite Edukt. Bevorzugt ist ein Verhältnis vom ersten Edukt zum zweiten Edukt im Bereich von eins zu drei mit einer Varianz von 5% oder von eins zu zwei mit einer Varianz von 5%. Der Begriff Anteil meint hier den Stoffmengenanteil in mol% des Feedgasstroms. Ein Verhältnis von eins zu drei mit einer Varianz von 5% ist insbesondere bei Kohlendioxid und Wasserstoff bevorzugt. Ein Verhältnis von eins zu zwei mit einer Varianz von 5% ist insbesondere bei Kohlenmonoxid und Wasserstoff bevorzugt.

Der Syntheseloop umfasst vorzugsweise die Synthesereaktoranordnung und alle Leitungen, die den Auslass der Synthesereaktoranordnung mit dem Einlass der Synthesereaktoranordnung verbinden.

Der Druck im Syntheseloop kann im Wesentlichen durch die Stoffmenge in der Gasphase, die Temperatur und das Syntheseloopvolumen bestimmt werden. Die Verringerung des Stoffmengenstroms in der Gasphase kann durch Reaktion des ersten Edukts und des zweiten Edukts zum Methanol in der Synthesereaktoranordnung und die Kondensation und Abscheidung des Methanols bedingt werden. Der Druckabfall des Syntheseloops kann umso höher sein, je mehr Produkt gebildet wird und abgeschieden wird, sofern nicht mit dem Feedgas die aus der Gasphase verlorene Stoffmenge wieder zugeführt wird.

Das erste Edukt weist vorzugsweise eine höhere Löslichkeit als das zweite Edukt in einem Methanol-Wasser-Gemisch auf, sodass der Anteil des ersten Edukts in der Gasphase durch eine Löslichkeit in diesem Methanol-Wasser-Gemisch noch weiter reduziert werden kann.

Die Kondensation des in der Synthesereaktoranordnung gebildeten Methanols kann in zwei Schritten erfolgen. Im ersten Schritt wird das Produktgasgemisch am Auslass der Synthesereaktoranordnung über einen Wärmeübertrager im Gegenstrombetrieb gekühlt. Gleichzeitig wird der Gasstrom am Einlass der Synthesereaktoranordnung über den Wärmeübertrager aufgewärmt. Im zweiten Schritt wird in einem oder mehreren luft- oder wassergekühlten Wärmeübertragern das Produktgasgemisch am Auslass der Synthesereaktoranordnung, inklusive teilkondensierten Methanols und unreagierter Edukte, auf die finale Temperatur des Abscheiders gekühlt, wobei weiteres Methanol kondensiert.

Die Reaktionsrate von dem ersten Edukt und dem zweiten Edukt zu Methanol kann in der Synthesereaktoranordnung bei konstanter Temperatur und konstanter Katalysatormenge durch die Partialdrücke des ersten Edukts und des zweiten Edukts bestimmt werden. Weiterhin kann aufgrund der Gleichgewichtsreaktion die Reaktionsrate auch durch den Partialdruck des Methanols und den Partialdruck eines oder mehrerer Nebenprodukte bestimmt werden.

Ein Wechsellastbetrieb bezeichnet den Betrieb der Synthesereaktoranordnung mit wechselnder Last, d.h. wechselndem Reaktoreintrittsgasstrom, insbesondere infolge eines wechselnden Eduktstroms der dem Syntheseloop zugeführt wird. Der sogenannte Lastbereich für den Eduktstrom der dem Syntheseloop zugeführt wird liegt vorzugsweise zu jedem Zeitpunkt des Verfahrens zwischen 0% und 100% der Anlagenkapazität, bevorzugt zwischen 20% und 100% der Anlagenkapazität. Eine Last von 100% wird auch als Volllast bezeichnet. Ein Lastbereich unter 100% wird auch als Teillast bezeichnet.

In zumindest einem ersten Fall wird die Stoffmengenströmungsrate des Feedgasstroms reduziert. Bei einem Lastwechsel von einem höheren Lastbereich in einen niedrigeren Lastbereich kann die Stoffmengenströmungsrate des Feedgasstroms abnehmen. Hierfür nimmt der Stoffmengenstrom des ersten Edukts und/oder der Stoffmengenstrom des zweiten Edukts ab.

Aufgrund der Trägheit der im Syntheseloop befindlichen Gasmenge und Zusammensetzung setzt die Synthesereaktoranordnung die Edukte zunächst weiterhin wie im höheren Lastbereich um. Mit der reduzierten Stoffmengenströmungsrate kann jedoch ein Defizit des ersten Edukts und/oder des zweiten Edukts entstehen und damit ein Druckabfall der Partialdrücke des ersten Edukts und/oder des zweiten Edukts.

Indem der Anteil des ersten Eduktes in dem Feedgasstrom reduziert wird, wird die Erzeugungsrate des Methanols zumindest zeitweise reduziert. Dies hat den Vorteil, dass der Partialdruck des ersten Edukts reduziert wird, wodurch die Reaktion gehemmt wird und die Erzeugungsrate von Methanol absinkt. Dies hat den weiteren Vorteil, dass die Stoffmenge in der Gasphase stabilisiert wird und der Druck im Syntheseloop nicht oder zumindest nur geringfügig abfällt. Da die Erzeugungsrate von Methanol reduziert wird, wird weniger vom ersten Edukt und weniger vom zweiten Edukt umgesetzt. Somit wird aufgrund der reduzierten Edukt-Stoffmengenströmungsrate pro Zeiteinheit weniger Methanol gebildet, wodurch der Druck in der Synthesereaktoranordnung aufgrund der Stöchiometrie der Methanolbildungsreaktion weniger stark abfällt. Das Defizit an erstem oder zweitem Edukt trägt dadurch zur Stabilisierung des Drucks in der Synthesereaktoranordnung bei.

Das erste Edukt macht vorzugsweise bei stöchiometrischem Verhältnis der Edukte stoffmengenmäßig einen geringeren Anteil im Feedgasstrom in den Syntheseloop und in die Synthesereaktoranordnung aus, im Vergleich zum zweiten Edukt. Eine Hemmung der Reaktion kann daher vergleichsweise einfach mit einer Verringerung des Anteils des ersten Eduktes erreicht werden. Dies hat den Vorteil, dass eine Verringerung des Stoffmengenanteils in Bezug auf die gesamte Stoffmenge an Edukten minimiert werden kann, was einen geringen Partialdruckabfall nach sich zieht und damit einen geringeren Einfluss auf den Gesamtdruckabfall ausübt.

Das beschriebene Verfahren ist vor allem dazu geeignet, auf vergleichsweise schnelle Schwankungen im Feedgasstrom zu reagieren. Auf langsamere Schwankungen kann auch auf andere Weise reagiert werden. Um gegenüber einem herkömmlichen Verfahren einen Vorteil zu erzielen, genügt es daher, dass in zumindest einem ersten Fall einer Reduzierung einer Stoffmengenströmungsrate des Feedgasstroms die Erzeugungsrate des Methanols zumindest zeitweise reduziert wird, indem ein Anteil des ersten Eduktes in dem Feedgasstrom reduziert wird. Dabei kann die Veränderung der Zufuhr am ersten Edukt auch schon durchgeführt werden, bevor ein Lastwechsel initiiert wird, d.h. bevor die Zufuhr an einem zweiten Edukt (H2 aus Elektrolyse) sich ändert. Auf das Verhältnis der Edukte im Feedgasstrom bei einer Vergrößerung der Stoffmengenströmungsrate des Feedgasstroms kommt es im Allgemeinen nicht an, um den Druck im Syntheseloop zu kontrollieren. Auch ist das Verhältnis der Edukte im Feedgasstrom bei konstantem Feedgasstrom unbeachtlich. Es genügt bereits, dass in zumindest einem ersten Fall einer (erwarteten) Reduzierung einer Stoffmengenströmungsrate des Feedgasstroms eine Erzeugungsrate des Methanols zumindest zeitweise reduziert wird, um die Auswirkungen der Verringerung der Stoffmengenströmungsrate des Feedgasstroms kurzfristig auszugleichen. Das liegt daran, dass der Druck in dem Rohrleitungssystem neben der Beeinflussung des Verhältnisses der Edukte im Feedgasstrom auch auf andere Arten beeinflusst werden kann. Hierzu kommen insbesondere alle Möglichkeiten in Betracht, welche auch bei einer herkömmlichen Anordnung zur Synthese von Methanol eingesetzt werden. Beispielsweise kann ein zu großer Druck über ein Überdruckventil abgelassen werden, welches vergleichsweise schnell reagieren kann. Die Gefahr eines zu großen Drucks unterscheidet sich also qualitativ von der Gefahr eines zu geringen Drucks.

Auch ist es nicht erforderlich, dass der Anteil des ersten Eduktes in dem Feedgasstrom jedes Mal reduziert wird, wenn sich die Stoffmengenströmungsrate des Feedgasstroms verringert. Es genügt, dass der Anteil des ersten Eduktes in dem Feedgasstrom zumindest in einem ersten Falle einer Verringerung der Stoffmengenströmungsrate des Feedgasstroms verringert wird, also mindestens einmal bei der Durchführung des Verfahrens. Insbesondere im Falle einer langsamen Verringerung der Stoffmengenströmungsrate des Feedgasstroms mag es nicht erforderlich sein, mit einer Verringerung des Anteils des ersten Edukts in dem Feedgasstrom zu reagieren. Es ist jedoch bevorzugt, dass der Anteil des ersten Eduktes in dem Feedgasstrom jedes Mal reduziert wird, wenn sich die Stoffmengenströmungsrate des Feedgasstroms schneller als ein Druckveränderungs-Grenzwert verringert und/oder jedes Mal reduziert wird, wenn sich die Stoffmengenströmungsrate des Feedgasstroms auf einen Wert unterhalb eines Druck-Grenzwert verringert.

In einer bevorzugten Ausführungsform wird in dem zumindest einen ersten Fall der Anteil des ersten Eduktes in dem Feedgasstrom reduziert, indem ein Puffergas zum Feedgasstrom hinzugefügt wird.

Die Reaktionsrate der Edukte zu Methanol kann reduziert werden, indem der Partialdruck der Edukte reduziert wird. Die Wahrscheinlichkeit für die Reaktion vom ersten Edukt und zweiten Edukt zu Methanol nimmt mit niedrigerem Partialdruck ab. Hinzufügen von einem Puffergas zum Feedgasstrom bei einem definierten Gesamtdruck reduziert den Anteil der Edukte im Feedgasstrom und somit deren Partialdruck.

Eine Reduzierung der Erzeugungsrate ist von Vorteil, da das erste Edukt und das zweite Edukt zu Methanol reagieren und die reagierte Stoffmenge der Gasphase des Syntheseloops entzogen wird und so einen Druckabfall begünstigt. Wenn nun weniger Methanol produziert wird, wird dementsprechend weniger Edukt verbraucht.

Diese Ausführungsform dient vorzugsweise dazu, den Partialdruck des ersten Edukts und des zweiten Edukts zu reduzieren, die Reaktion zu hemmen und weniger Methanol herzustellen. Ein weiterer Vorteil dieser Ausführungsform ist, dass das Puffergas einen Druckabfall stabilisieren kann, indem das Puffergas zur Druckhaltung im Syntheseloop fehlende Stoffmenge der Gasphase auffüllen kann und der Gesamtdruck im Syntheseloop stabilisiert wird.

In einer weiteren bevorzugten Ausführungsform wird in dem zumindest einen ersten Fall der Anteil des ersten Eduktes in dem Feedgasstrom reduziert, indem eine Menge des ersten Edukts in dem Feedgasstrom relativ zu einer Menge des zweiten Edukts in dem Feedgasstrom reduziert wird.

Dies kann vorzugsweise erreicht werden, indem das zweite Edukt überstöchiometrisch dem Feedgasstrom hinzugefügt wird.

Vorzugsweise fluktuiert die Menge des zweiten Edukts. Eine Fluktuation meint die Änderung des Stoffmengenstroms und/oder des Partialdrucks des zweiten Edukts über die Zeit.

Diese Ausführungsform dient vorzugsweise dazu, die Erzeugungsrate des Methanols zu reduzieren, indem der Partialdruck des ersten Edukts so weit reduziert wird, dass die Reaktion gehemmt wird.

In einer ersten Möglichkeit kann die Stoffmengenströmungsrate des zweiten Eduktes erhöht werden. So kann die Reaktion durch eine Verknappung an erstem Edukt gehemmt werden, und der Überschuss an zweitem Edukt gleicht die Verknappung der Stoffmenge an einem ersten Edukt aus. Der Druck im Syntheseloop kann so stabilisiert werden. Nachteil dieser Möglichkeit ist, dass das zweite Edukt nicht ohne weiteres zur Verfügung steht. Denn in dem zumindest einen ersten Fall kann der Feedgasstrom insbesondere dann reduziert werden, wenn das zweite Edukt zeitweise weniger zur Verfügung steht.

In einer zweiten Möglichkeit wird die Stoffmengenströmungsrate des ersten Eduktes reduziert, bevor das zweite Edukt durch Fluktuation absinkt. Dies hat den Effekt, dass das zweite Edukt überstöchiometrisch im Feedgasstrom vorliegt. Durch Verringerung der Menge des ersten Edukts reagiert das immer weniger werdende erste Edukt mit dem zweiten Edukt zu Methanol, wodurch der Anteil des ersten Edukts im Syntheseloop sinkt. Hierdurch sinkt der Partialdruck des ersten Edukts ab und die Reaktion wird gehemmt. Zugleich reichert sich der Gehalt an einem zweiten Edukt im Syntheseloop an. Steht nun durch die eintretende Fluktuation weniger zweites Edukt zur Verfügung, dann kann der Feedgasstrom reduziert werden und der Druck im Syntheseloop ist aufgrund der gehemmten Reaktion stabil.

Vorzugsweise kann der Anteil des ersten Eduktes in dem Feedgasstrom in dem zumindest einen ersten Fall so weit reduziert werden, dass das erste Edukt im Syntheseloop unterstöchiometrisch vorliegt.

Den vorhergehenden Ausführungsformen ist gemein, dass in zumindest einem ersten Fall einer Reduzierung einer Stoffmengenströmungsrate des Feedgasstroms eine Erzeugungsrate des Methanols reduziert wird, indem der Anteil des ersten Edukts die Reaktion zu Methanol durch einen niedrigen Partialdruck hemmt.

In einer weiteren bevorzugten Ausführungsform wird Methanol aus einem Austrittsstrom der Synthesereaktoranordnung abgeschieden und der verbleibende Austrittsstrom der Synthesereaktoranordnung als ein Recyclegasstrom zumindest teilweise erneut zugeführt, wobei in dem zumindest einen ersten Fall ein Verhältnis einer Stoffmengenströmungsrate des Recyclegasstroms zu einer Stoffmengenströmungsrate des Feedgasstroms erhöht wird.

Das Verhältnis der Stoffmengenströmungsrate des Recyclegasstroms zu der Stoffmengenströmungsrate des Feedgasstroms wird auch Recycleverhältnis genannt.

Vorzugsweise ist in dem zumindest einen ersten Fall ein Recycleverhältnis von 2 bis 200 vorhanden. Bevorzugt ist ein Recycleverhältnis von 3 bis 30.

Vorzugsweise weisen der gemischte Feedgasstrom und Recyclegasstrom am Einlass der Synthesereaktoranordnung ein Stoffmengenverhältnis vom ersten Edukt zum zweiten Edukt im Bereich von 0,001 bis 0,33auf, bevorzugt im Bereich von 0,005 bis 0,33.

Eine signifikante Erhöhung des Recycleverhältnisses und somit verstärkte Anreicherung nichtreagierter Komponenten ist im Allgemeinen nur im Teillastbereich möglich, da das maximale Recycleverhältnis durch die Obergrenze eines förderbaren Recyclegasstroms limitiert ist.

Diese Ausführungsform dient vorzugsweise dazu, den Umsatz der Edukte zu erhöhen und Inertgase sowie unreagierte Edukte als Puffer zu akkumulieren.

Das erste Edukt und das zweite Edukt können im Einlass der Synthesereaktoranordnung und/oder vor dem Einlass der Synthesereaktoranordnung gemischt werden. Weitere Ströme wie der Recyclegasstrom und das Puffergas können dem Feedgasstrom im Einlass und/oder vor dem Einlass der Synthesereaktoranordnung beigemischt werden.

In zumindest einem zweiten Fall wird die Stoffmengenströmungsrate des Feedgasstroms vorzugsweise erhöht. Analog zum zumindest einen ersten Fall ist es nicht erforderlich, dass der Anteil des ersten Eduktes in dem Feedgasstrom jedes Mal erhöht wird, wenn sich die Stoffmengenströmungsrate des Feedgasstroms vergrößert. Es genügt, dass der Anteil des ersten Eduktes in dem Feedgasstrom in zumindest einem zweiten Falle einer Vergrößerung der Stoffmengenströmungsrate des Feedgasstroms vergrößert wird, also mindestens einmal bei der Durchführung des Verfahrens.

In einer weiteren bevorzugten Ausführungsform wird in zumindest einem zweiten Fall einer Erhöhung einer Stoffmengenströmungsrate des Feedgasstroms eine Erzeugungsrate des Methanols zumindest zeitweise erhöht, indem der Anteil des ersten Eduktes in dem Feedgasstrom erhöht wird.

Vorzugsweise wird ein etwaiger Druckanstieg im Syntheseloop durch die Erhöhung der Stoffmengenströmungsrate über eine Spülgasleitung abgelassen. Besonders bevorzugt wird der Austrittsstrom der Synthesereaktoranordnung als Spülgas zumindest teilweise entfernt. Dadurch kann das zweite Edukt, welches überstöchiometrisch im Syntheseloop vorhanden ist und/oder das Puffergas aus dem Syntheseloop entfernt werden. Der Partialdruck des ersten Edukts wird mit neu in den Loop eingespeister Menge an erstem Edukt erhöht und hemmt nicht mehr die Reaktion. Die erhöhte Stoffmengenströmungsrate des Feedgasstroms erhöht dann entsprechend die Erzeugungsrate des Methanols.

Diese Ausführungsform dient vorzugsweise dazu, die Erzeugungsrate des Methanols in dem zumindest einen zweiten Fall zügig zu erhöhen und den Gesamtdruck in der Synthesereaktoranordnung nicht ansteigen zu lassen.

In einer weiteren bevorzugten Ausführungsform wird in dem zumindest einen zweiten Fall der Anteil des ersten Eduktes in dem Feedgasstrom erhöht, indem eine Menge des ersten Edukts in dem Feedgasstrom relativ zu einer Menge des zweiten Edukts in dem Feedgasstrom erhöht wird.

Durch den ersten Fall einer Reduzierung der Stoffmengenströmungsrate kann das zweite Edukt überstöchiometrisch im Syntheseloop vorhanden sein.

Wenn der Feedgasstrom rapide zunimmt, kann die Erzeugungsrate des Methanols zunächst zu niedrig sein. Die Folge kann ein steigender Druck im Syntheseloop sein. Um die Erzeugungsrate des Methanols wieder zu erhöhen, wird der Anteil des ersten Eduktes im Feedgasstrom zumindest zeitweise erhöht. Der Partialdruck des ersten Edukts steigt und hemmt nicht mehr die Erzeugungsrate des Methanols. Dieser erhöhte Anteil des ersten Edukts kann mit dem überstöchiometrisch vorhandenen zweiten Edukt reagieren und die Erzeugungsrate des Methanols erhöhen, sodass das erste Edukt und das zweite Edukt zu Methanol reagieren. Dies hat den Effekt, dass der überschüssige Stoffmengenstrom durch den erhöhten Feedgasstrom durch die Reaktion von ersten Edukt und zweiten Edukt zu Methanol abgebaut wird.

Diese Ausführungsform dient vorzugsweise dazu, die Erzeugungsrate des Methanols in dem zumindest einen zweiten Fall zu erhöhen und den Gesamtdruck im Syntheseloop nicht ansteigen zu lassen.

Vorzugsweise ist das erste Edukt Kohlenstoffdioxid oder Kohlenstoffmonoxid.

Vorzugsweise ist das zweite Edukt Wasserstoff.

Besonders bevorzugt stammt das zweite Edukt aus einer Wasserelektrolyse mit erneuerbarer Energie.

Der Feedgasstrom umfasst vorzugsweise 0 mol% bis 35 mol% Kohlenstoffdioxid und 65 mol% bis 100 mol% Wasserstoff. Bevorzugt umfasst der Feedgasstrom 15 mol% bis 30 mol% Kohlenstoffdioxid und 70 mol% bis 85 mol% Wasserstoff.

In einer weiteren bevorzugten Ausführungsform enthält das Puffergas ein Inertgas oder mehrere Inertgase und/oder Methan und/oder Wasserstoff.

Das Inertgas ist vorzugsweise Argon. Das Inertgas ist besonders bevorzugt Stickstoff. Es können auch mehre Inertgase gemischt werden. Das Inertgas beinhaltet vorzugsweise ein Gemisch aus Argon und/oder Stickstoff.

In einer weiteren bevorzugten Ausführungsform fluktuiert ein Gesamtdruck in der Synthesereaktoranordnung während der Durchführung des Verfahrens um nicht mehr als 40 %, bevorzugt nicht mehr als 20 %, bevorzugt nicht mehr als 10%.

Ein erwünschter Gesamtdruck ist ein vom Lastbereich unabhängiger Betriebsdruck der Synthesereaktoranordnung. Hohe Druckschwankungen erhöhen die Anforderungen an die verwendeten Rohrleitungen und Apparate, da diese für Druckschwankungen ausgelegt sein müssen.

Das beschriebene Verfahren ist besonders dann sinnvoll, wenn die Synthese von Methanol im Wechsellastbetrieb ermöglicht werden soll, vor allem wenn der Feedgasstrom reduziert werden soll. Dieser Fall kann eintreten, wenn das zweite Edukt fluktuiert. Statt den reduzierten Edukt-Stoffmengenstrom durch ein Puffergas vollständig aufzufüllen, um einen Druckabfall zu verhindern, kann durch Reduzierung des ersten Edukts-Stoffmengenstroms die Reaktion zu Methanol gehemmt werden. Besonders vorteilhaft ist es, den ersten Edukt-Stoffmengenstrom zu reduzieren, bevor der zweite Edukt-Stoffmengenstrom durch Fluktuation reduziert wird.

Als ein weiterer Aspekt der Erfindung wird eine Vorrichtung zur Synthese von Methanol vorgestellt, welche eine Synthesereaktoranordnung, eine erste Eduktgasquelle für ein erstes Edukt, eine zweite Eduktgasquelle für ein zweites Edukt und eine Steuereinrichtung umfasst, wobei die erste Eduktgasquelle und die zweite Eduktgasquelle mit einem Einlass der Synthesereaktoranordnung verbunden sind, und wobei die Steuereinrichtung dazu eingerichtet ist, die Vorrichtung mit dem beschriebenen Verfahren zu betreiben.

Die erste Eduktgasquelle kann vorzugsweise ein Kohlenstoffdioxid-Abscheideprozess oder eine Kohlenstoffdioxidleitung sein. Das erste Edukt ist vorzugsweise Kohlenstoffdioxid und/oder Kohlenstoffmonoxid.

Die zweite Eduktgasquelle ist vorzugsweise ein Wasserelektrolyseprozess. Der Wasserelektrolyseprozess wird vorzugsweise mit einer Versorgung von erneuerbarer Energie betrieben, welche fluktuieren kann. Das zweite Edukt ist vorzugsweise Wasserstoff.

Für die Steuereinrichtung wird vorzugsweise die Gaszusammensetzung, der Stoffmengenstrom und der Druck an verschiedenen Stellen gemessen. Insbesondere im Feedgasstrom wird vorzugsweise untersucht, welcher Anteil des ersten Edukts, des zweiten Edukts und etwaiger Restkomponenten wie beispielsweise Inertgase vor Eintritt in den Syntheseloop vorliegen. Weitere Messstellen können am Einlass der Synthesereaktoranordnung oder am Auslass der Synthesereaktoranordnung vorgesehen sein. Falls ein Recyclegasstrom vorhanden ist, kann in der Recycleleitung die Gaszusammensetzung, der Mengenstrom und der Druck gemessen werden.

In einer weiteren bevorzugten Ausführungsform weist die Vorrichtung weiterhin eine Puffergasquelle für ein Puffergas auf, wobei die Puffergasquelle mit einem Einlass der Synthesereaktoranordnung verbunden ist.

Diese Ausführungsform dient vorzugsweise dazu, in dem zumindest einen ersten Fall den Anteil des ersten Eduktes in dem Feedgasstrom zu reduzieren, indem ein Puffergas zum Feedgasstrom hinzugefügt wird.

In einer weiteren bevorzugten Ausführungsform umfasst die Vorrichtung weiterhin eine Recycleleitung, über welche ein Auslass der Synthesereaktoranordnung mit einem Einlass der Synthesereaktoranordnung verbunden ist.

In dieser bevorzugten Ausführungsform kann der Umsatz der Edukte zu Methanol erhöht werden und Inertgase sowie unreagierte Edukte als Puffer akkumuliert werden.

In einer weiteren bevorzugten Ausführungsform umfasst die Vorrichtung weiterhin eine Spülgasleitung, welche von der Recycleleitung abzweigt.

In dieser bevorzugten Ausführungsform kann ein erhöhter Druck im Syntheseloop als Reaktion auf einen spontan angestiegenen Feedgasstrom kontrolliert in einem definierten Bereich gehalten bzw. gesenkt werden.

Die Verwendung einer wie beschrieben ausgestalteten Vorrichtung hat den Vorteil, dass ein Wechsellastbetrieb in der Synthese von Methanol ermöglicht wird. Der Wechsellastbetrieb kann durch eine Reduzierung und Erhöhung des Feedgasstroms eintreten und auf einem fluktuierenden zweiten Edukt basieren. Durch eine Reduzierung und Erhöhung des Anteils des ersten Edukts im Feedgasstrom kann die Reaktionsrate gesteuert werden und dadurch kann der Gesamtdruck im Syntheseloop kontrolliert werden.

Die Erfindung wird nachfolgend anhand der Figur näher erläutert. Die Figur zeigt ein besonders bevorzugtes Ausführungsbeispiel, auf welches die Erfindung jedoch nicht begrenzt ist. Die Figur und die darin dargestellten Größenverhältnisse sind nur schematisch. Es zeigen:

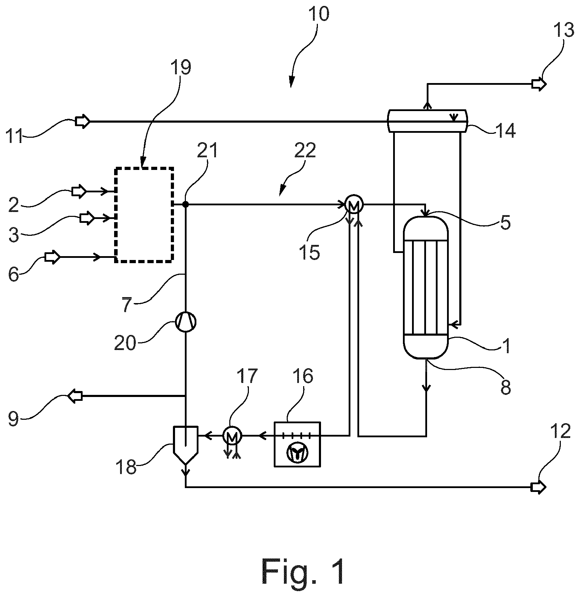

- Fig. 1:: eine schematische Darstellung einer erfindungsgemäßen Vorrichtung zur Synthese von Methanol.

Fig. 1 zeigt eine schematische Darstellung einer erfindungsgemäßen Vorrichtung 10 zur Synthese von Methanol, welche eine Synthesereaktoranordnung 1, eine erste Eduktgasquelle 2 für ein erstes Edukt, eine zweite Eduktgasquelle 3 für ein zweites Edukt, eine Puffergasquelle 6 für ein Puffergas und eine nicht gezeigte Steuereinrichtung umfasst, wobei die erste Eduktgasquelle 2, die zweite Eduktgasquelle 3 und die Puffergasquelle 6 mit einem Einlass 5 der Synthesereaktoranordnung 1 verbunden sind. Das erste Edukt, das zweite Edukt und das Puffergas, sofern ein Puffergas eingebracht wird, werden über einen ersten Verdichter 19 verdichtet. Das erste Edukt ist Kohlenstoffdioxid. Das zweite Edukt ist Wasserstoff, welches aus einem Elektrolyseprozess mit erneuerbarer Energie stammt.

Die Vorrichtung 10 umfasst weiterhin eine Recycleleitung 7, über welche ein Auslass 8 der Synthesereaktoranordnung 1 mit dem Einlass 5 der Synthesereaktoranordnung 1 verbunden ist. Die Recyclingleitung 7 umfasst einen Wärmeübertrager 15, einen Syntheseluftkühler 16, einen finalen Wasserkühler 17 und einen Abscheider 18. Ein Syntheseloop 22 umfasst die Synthesereaktoranordnung 1, die Recycleleitung 7 sowie eine Leitung von einer Mischstelle 21 bis zum Einlass 5 der Synthesereaktoranordnung 1.

Die Synthesereaktoranordnung 1 produziert Methanol und Wasserdampf aus Kohlenstoffdioxid und Wasserstoff. Die Kondensation des in der Synthesereaktoranordnung 1 gebildeten Methanols erfolgt in zwei Schritten. Im ersten Schritt wird das Produktgasgemisch am Auslass der Synthesereaktoranordnung 1 über den Wärmeübertrager 15 im Gegenstrombetrieb gekühlt. Gleichzeitig wird der Gasstrom des Syntheseloops 22 aufgewärmt. Der Syntheseloopstrom enthält den Feedgasstrom und den Recyclegasstrom. Im zweiten Schritt wird im Syntheseluftkühler 16 und im finalen Wasserkühler 17 das Produktgasgemisch am Auslass der Synthesereaktoranordnung 1 sowie das teilkondensierte Methanol und die unreagierten Edukte auf die finale Abscheidetemperatur gekühlt. Im Abscheider 18 wird flüssiges Methanol vom Gasstrom getrennt. Das Methanol wird an einem Methanolauslass 12 ausgegeben.

Eine Spülgasleitung 9 zweigt von der Recycleleitung 7 ab. Der Recyclegasstrom in der Recycleleitung 7 wird in einem zweiten Verdichter 20 auf einen Betriebsdruck verdichtet. Der Feedgasstrom und der Puffergasstrom werden in der Mischstelle 21 mit dem Recyclegasstrom vermischt.

Eine Frischwasserquelle 11 liefert Wasser an einen Dampfkessel 14. Dieser produziert Wasserdampf 13 und heißes Wasser und/oder gesättigten Dampf. Die Temperatur der Synthesereaktoranordnung 1 kann während der Fluktuation des Feedgasstroms konstant gehalten werden, entweder durch Kühlung der Reaktionswärme und Verdampfung von Wasser auf der Mantelseite der Synthesereaktoranordnung 1 oder durch Erwärmung bei unzureichender Reaktionsabwärme durch zusätzliche Zufuhr von gesättigtem Dampf oder heißem Wasser auf der Mantelseite aus dem Dampfkessel 14.

Die Steuereinrichtung ist dazu eingerichtet, die Vorrichtung 10 mit dem folgenden Verfahren zur Synthese von Methanol zu betreiben.

Ein Feedgasstrom umfasst Kohlenstoffdioxid und Wasserstoff und wird der Synthesereaktoranordnung 1 zugeführt, wobei in der Synthesereaktoranordnung 1 mit Kohlenstoffdioxid und Wasserstoff Methanol erzeugt wird, wobei in zumindest einem ersten Fall einer Reduzierung einer Stoffmengenströmungsrate des Feedgasstroms eine Erzeugungsrate des Methanols zeitweise reduziert wird, indem ein Anteil des Kohlenstoffdioxids in dem Feedgasstrom reduziert wird.

In dem zumindest einen ersten Fall wird der Anteil des Kohlenstoffdioxids in dem Feedgasstrom reduziert, indem ein Puffergas aus Stickstoff und Wasserstoff zum Feedgasstrom hinzugefügt wird. Weiterhin wird in dem zumindest einen ersten Fall der Anteil des Kohlenstoffdioxids in dem Feedgasstrom reduziert, indem eine Menge des Kohlenstoffdioxids in dem Feedgasstrom relativ zu einer Menge des Wasserstoffs in dem Feedgasstrom reduziert wird. Dabei wird der Anteil des Kohlenstoffdioxids in dem Feedgasstrom so reduziert, dass das Kohlenstoffdioxid in dem Feedgasstrom unterstöchiometrisch vorliegt.

Das Methanol aus einem Austrittsstrom eines Auslasses 8 der Synthesereaktoranordnung 1 wird abgeschieden und der verbleibende Austrittsstrom der Synthesereaktoranordnung 1 als ein Recyclegasstrom teilweise erneut zugeführt, und wobei in dem zumindest einen ersten Fall ein Verhältnis einer Stoffmengenströmungsrate des Recyclegasstroms zu einer Stoffmengenströmungsrate des Feedgasstroms erhöht wird. Das Recycleverhältnis beträgt in dem zumindest einen ersten Fall beispielsweise 20.

In zumindest einem zweiten Fall einer Erhöhung einer Stoffmengenströmungsrate des Feedgasstroms wird die Erzeugungsrate des Methanols zeitweise erhöht, indem der Anteil des Kohlenstoffdioxids in dem Feedgasstrom erhöht wird. Hierfür wird der Austrittsstrom der Synthesereaktoranordnung 1 als Spülgas teilweise entfernt.

Der Anteil des Kohlenstoffdioxids in dem Feedgasstrom wird erhöht, indem eine Menge des Kohlenstoffdioxids in dem Feedgasstrom relativ zu einer Menge des Wasserstoffs in dem Feedgasstrom erhöht wird.

Der Gesamtdruck in der Synthesereaktoranordnung 1 fluktuiert während der Durchführung des Verfahrens um nicht mehr als 10 %.

### Bezugszeichen

- 1: Synthesereaktoranordnung
- 2: erste Eduktgasquelle
- 3: zweite Eduktgasquelle
- 5: Einlass des Synthesereaktoranordnung
- 6: Puffergasquelle
- 7: Recycleleitung
- 8: Auslass des Synthesereaktoranordnung
- 9: Spülgasleitung
- 10: Vorrichtung
- 11: Frischwasserquelle
- 12: Methanolauslass
- 13: Wasserdampf
- 14: Dampfkessel
- 15: Wärmeübertrager
- 16: Syntheseluftkühler
- 17: Wasserkühler
- 18: Abscheider
- 19: erster Verdichter
- 20: zweiter Verdichter
- 21: Mischstelle
- 22: Syntheseloop

## Patentansprüche

1. Verfahren zur Synthese von Methanol, wobei ein Feedgasstrom umfassend zumindest ein erstes Edukt und ein zweites Edukt einer Synthesereaktoranordnung (1) zugeführt wird, wobei in der Synthesereaktoranordnung (1) mit dem ersten Edukt und dem zweiten Edukt Methanol erzeugt wird, wobei in zumindest einem ersten Fall einer Reduzierung einer Stoffmengenströmungsrate des Feedgasstroms eine Erzeugungsrate des Methanols zumindest zeitweise reduziert wird, indem ein Anteil des ersten Eduktes in dem Feedgasstrom reduziert wird.

2. Verfahren nach Anspruch 1, wobei in dem zumindest einen ersten Fall der Anteil des ersten Eduktes in dem Feedgasstrom reduziert wird, indem ein Puffergas zum Feedgasstrom hinzugefügt wird.

3. Verfahren nach einem der vorhergehenden Ansprüche, wobei in dem zumindest einen ersten Fall der Anteil des ersten Eduktes in dem Feedgasstrom reduziert wird, indem eine Menge des ersten Edukts in dem Feedgasstrom relativ zu einer Menge des zweiten Edukts in dem Feedgasstrom reduziert wird.

4. Verfahren nach einem der vorhergehenden Ansprüche, wobei in dem zumindest einen ersten Fall ein Anteil des ersten Eduktes in dem Feedgasstrom reduziert wird, sodass das erste Edukt in dem Feedgasstrom unterstöchiometrisch vorliegt.

5. Verfahren nach einem der vorhergehenden Ansprüche, wobei Methanol aus einem Austrittsstrom der Synthesereaktoranordnung (1) abgeschieden wird und der verbleibende Austrittsstrom der Synthesereaktoranordnung (1) als ein Recyclegasstrom zumindest teilweise erneut zugeführt wird, und wobei in dem zumindest einen ersten Fall ein Verhältnis einer Stoffmengenströmungsrate des Recyclegasstroms zu einer Stoffmengenströmungsrate des Feedgasstroms erhöht wird.

6. Verfahren nach einem der vorhergehenden Ansprüche, wobei in zumindest einem zweiten Fall einer Erhöhung einer Stoffmengenströmungsrate des Feedgasstroms eine Erzeugungsrate des Methanols zumindest zeitweise erhöht wird, indem der Anteil des ersten Eduktes in dem Feedgasstrom erhöht wird.

7. Verfahren nach Anspruch 6, wobei in dem zumindest einen zweiten Fall der Anteil des ersten Eduktes in dem Feedgasstrom erhöht wird, indem eine Menge des ersten Edukts in dem Feedgasstrom relativ zu einer Menge des zweiten Edukts in dem Feedgasstrom erhöht wird.

8. Verfahren nach einem der vorhergehenden Ansprüche, wobei das erste Edukt Kohlenstoffdioxid und/oder Kohlenstoffmonoxid ist.

9. Verfahren nach einem der vorhergehenden Ansprüche, wobei das zweite Edukt Wasserstoff ist.

10. Verfahren nach Anspruch 9, wobei das zweite Edukt aus einer Wasserelektrolyse mit erneuerbarer Energie stammt.

11. Verfahren nach einem der Ansprüche 2 bis 10, wobei das Puffergas ein Inertgas oder mehrere Inertgase, und/oder Methan, und/oder Wasserstoff enthält.

12. Verfahren nach einem der Ansprüche 1 bis 11, wobei ein Gesamtdruck in der Synthesereaktoranordnung (1) während der Durchführung des Verfahrens um nicht mehr als 40 %, bevorzugt nicht mehr als 20 %, bevorzugt nicht mehr als 10 % fluktuiert.

13. Vorrichtung (10) zur Synthese von Methanol, umfassend eine Synthesereaktoranordnung (1), eine erste Eduktgasquelle (2) für ein erstes Edukt, eine zweite Eduktgasquelle (3) für ein zweites Edukt, und eine Steuereinrichtung, wobei die erste Eduktgasquelle (2) und die zweite Eduktgasquelle (3) mit einem Einlass (5) der Synthesereaktoranordnung (1) verbunden sind, und wobei die Steuereinrichtung dazu eingerichtet ist, die Vorrichtung mit einem Verfahren nach einem der Ansprüche 1 bis 12 zu betreiben.

14. Vorrichtung (10) nach Anspruch 13, wobei die Vorrichtung (10) weiterhin eine Puffergasquelle (6) für ein Puffergas aufweist, wobei die Puffergasquelle (6) mit einem Einlass (5) der Synthesereaktoranordnung (1) verbunden ist.

15. Vorrichtung (10) nach Anspruch 13 oder 14, wobei die Vorrichtung (10) weiterhin eine Recycleleitung (7) umfasst, über welche ein Auslass (8) der Synthesereaktoranordnung (1) mit einem Einlass (5) der Synthesereaktoranordnung (1) verbunden ist.
